# EUROPEAN PATENT SPECIFICATION

(11) **EP 2 574 312 B1**
(45) Date of publication and mention of the grant of the patent: **18.11.2015**
(21) Application number: 12186701.4
(22) Date of filing: 28.09.2012
(51) Int. Cl.: A61F 2/38, A61F 2/30, A61F 2/46

(54) **Tibial component of a knee prosthesis having an angled cement pocket**
Schienbeinkomponente einer Knieprothese mit angewinkelter Zementtasche
Composant tibial de prothèse du genou comportant une poche de ciment inclinée

(30) Priority: 30.09.2011 US 201113249496
(43) Date of publication of application: 03.04.2013
(60) Divisional application: 14185322.6
(73) Proprietor: DePuy (Ireland), County Cork (IE)
(72) Inventor: Wagner, Christel M., Warsaw, IN Indiana IN 46581 (US); Wyss, Joseph G., Warsaw, IN Indiana IN 46581 (US); Barrett, David S., Warsaw, IN Indiana IN 46581 (US)
(74) Representative: Belcher, Simon James

(56) References cited:
- EP-A2- 0 941 719
- WO-A1-2010/039963
- WO-A2-2005/082039
- DE-A1-102004 056 713
- US-A1- 2003 220 697
- US-A1- 2007 010 890

## Description

The present invention relates generally to an implantable orthopaedic prosthesis, and more particularly to an implantable knee prosthesis.

During the lifetime of a patient, it may be necessary to perform a joint replacement procedure on the patient as a result of, for example, disease or trauma. The joint replacement procedure may involve the use of a prosthesis which is implanted into one or more of the patient's bones. In the case of a knee replacement procedure, a tibial tray is implanted into the patient's tibia. A bearing is secured to the tibial tray. The condyle surfaces of a replacement femoral component bear against the tibial bearing.

During implantation of the femoral component, the surgeon typically preloads bone cement on the bone-contacting surfaces of the component. The preloaded bone cement has a tendency to escape the femoral component as it is positioned on the surgically-prepared distal femur. Advancement of the femoral component through escaped bone cement is referred to as "cement plowing". Both the escaped bone cement and the associated cement plowing lead to the performance of additional surgical steps to remove the extraneous bone cement prior to completion of the surgical procedure.

US-A-2007/010890 forms the basis for the preamble of claim 1. This document discloses a tibial component of a knee joint prosthesis, which comprises a modular base plate having an outer peripheral wall on its inferior surface which defines medial and lateral cavities on the inferior surface, and a stem extending from the inferior surface.

The present invention provides an implantable orthopaedic knee prosthesis comprising a tibial component, as defined in claim 1.

The implantable orthopaedic knee prosthesis may also include a tibial bearing component. In such a case, the tibial component may be embodied as a tibial tray, with the tibial bearing component being positioned on the tibial tray. The tibial tray may be metallic and the tibial bearing may be polymeric.

In other embodiments, the superior side of the tibial component may include a medial bearing surface configured to articulate with the medial condyle surface of a femoral component, and a lateral bearing surface configured to articulate with the lateral condyle surface of the femoral component. In such a case, the tibial component may be embodied as a monolithic polymeric component.

The inferior fixation surface of the tibial component may include a mounting rim having a sidewall extending superiorly therefrom. The sidewall defines the perimeter of the posterior cement pocket. When viewed sagittally, an anterior end of the sidewall is wider than a posterior end of the sidewall.

When viewed sagittally, an anterior end of the sidewall is wider than a posterior end of the sidewall.

The sidewall and the top wall collectively define a cement pocket.

The implantable orthopaedic knee prosthesis may also include a tibial bearing component. In such a case, the tibial component may be embodied as a tibial tray, with the tibial bearing component being positioned on the tibial tray. The tibial tray may be metallic and the tibial bearing may be polymeric.

In other embodiments, the superior side of the tibial component may include a medial bearing surface configured to articulate with the medial condyle surface of a femoral component, and a lateral bearing surface configured to articulate with the lateral condyle surface of the femoral component. In such a case, the tibial component may be embodied as a monolithic polymeric component.

The implantable orthopaedic knee prosthesis can include a femoral component having a medial condyle surface and a lateral condyle surface, and a bearing having (i) a medial bearing surface configured to articulate with the medial condyle surface of the femoral component, and (ii) a lateral bearing surface configured to articulate with the lateral condyle surface of the femoral component, with the tibial component is positioned below the bearing.

The tibial tray may be metallic and the tibial bearing may be polymeric.

In an embodiment the tibial bearing is rotatable relative to the tibial tray.

In another embodiment, the tibial bearing is fixed in position relative to the tibial tray.

The detailed description particularly refers to the following figures, in which:
FIG. 1 is a perspective view of a knee prosthesis;
FIG. 2 is an exploded perspective view of the knee prosthesis of FIG. 1;
FIG. 3 is a perspective view of the femoral component of the knee prosthesis of FIG. 1;
FIG. 4 is a sagittal cross section view of the femoral component of FIG. 3;
FIG. 5 is an enlarged, fragmentary view of a portion of FIG. 4 showing the posterior condyles of the femoral component in greater detail;
FIG. 6 is a view similar to FIG. 4, but showing another femoral component in which both the posterior and anterior cement pockets are angled;
FIG. 7 is a perspective view of a unicompartmental femoral component;
FIG. 8 is a sagittal cross sectional view of the unicompartmental femoral component of FIG. 7;
FIG. 9 is a plan view of the inferior side of the tibial tray of the knee prosthesis of FIG. 1;
FIG. 10 is a sagittal cross section view of the tibial tray taken along the line 10-10 of FIG. 9, as viewed in the direction of the arrows;
FIG. 11 is a sagittal cross section view of the tibial tray taken along the line 11-11 of FIG. 9, as viewed in the direction of the arrows; and
FIG. 12 is cross section view of an all-poly tibial component.

Terms representing anatomical references, such as anterior, posterior, medial, lateral, superior, inferior, etcetera, may be used throughout this document in reference to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

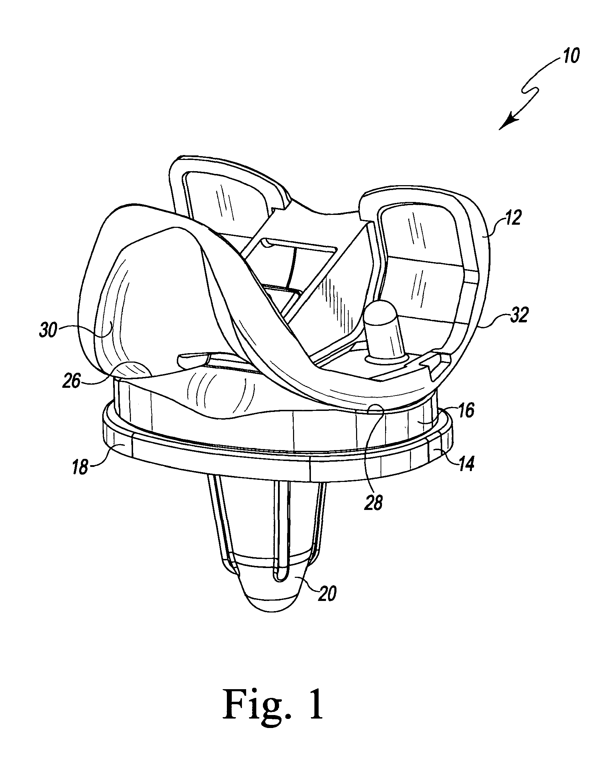

Referring now to FIGS. 1 to 5, there is shown a knee prosthesis 10. The knee prosthesis 10 includes a femoral component 12, a tibial tray 14, and a bearing 16. The femoral component 12 is configured to be secured to a surgically-prepared end of a patient's distal femur (not shown), whereas the tibial tray 14 is configured to be secured to a surgically-prepared end of a patient's proximal tibia (not shown).

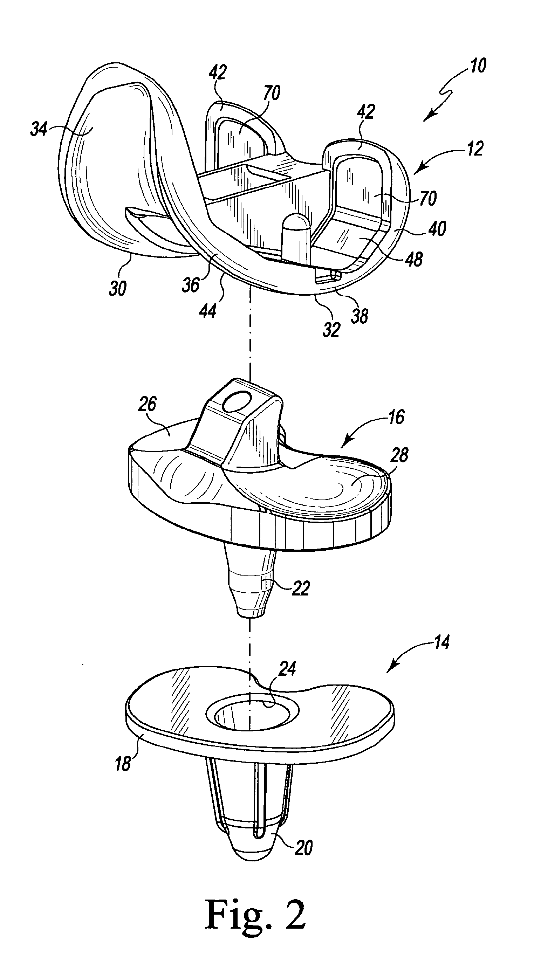

The tibial tray 14 includes a platform 18 having a fixation member, such as an elongated stem 20, extending away from its lower surface. The bearing 16 includes a stem 22 (see FIG. 2) that is positionable within a complementary bore 24 (see FIG. 2) in the tibial tray 14. In such a way, the bearing 16 is free to rotate relative to the tibial tray 14. In other devices, the bearing 16 may be snap-fit or otherwise secured to the tibial tray 14. In such a way, the bearing 16 is fixed relative to the tibial tray 14 (i.e., it is not rotative or moveable in the anterior/posterior or medial/lateral directions). It should be appreciated that in such devices, other fixation members, such as one or more short pegs or posts, may be used in lieu of the elongated stem 20.

The bearing 16 includes a lateral articular surface 26 and a medial articular surface 28. The articular surfaces 26, 28 are configured to articulate with a lateral condyle surface 30 and a medial condyle surface 32, respectively, of the femoral component 12. Specifically, the femoral component 12 is configured to emulate the configuration of the patient's natural femoral condyles, and, as such, the lateral condyle surface 30 and the medial condyle surface 32 are configured (e.g., curved) in a manner which mimics the condyles of the natural femur. The lateral condyle surface 30 and the medial condyle surface 32 are spaced apart from one another thereby defining an intercondylar notch therebetween.

The components of the knee prosthesis 10 that engage the natural bone, such as the femoral component 12 and the tibial tray 14, may be constructed with a biocompatible metal, such as a cobalt chrome alloy, although other materials, such as ceramics, may also be used. The bone engaging surfaces of these components may be textured to facilitate cementing the component to the bone. Such surfaces may also be porous coated to promote bone ingrowth for permanent fixation.

The bearing 16 may be constructed with a material that allows for smooth articulation between the bearing 16 and the femoral component 12, such as a polymeric material. One such polymeric material is polyethylene such as ultrahigh molecular weight polyethylene (UHMWPE), although other biocompatible polymers may be used.

Although the femoral component 12 is herein illustratively described as a monolithic component, it is characterized by a number of "regions" or "structures". For example, the anterior structure of the femoral component 12 is referred to as an anterior flange 34. The anterior flange 34 transitions to an anterior chamfer region 36, which, in turn, transitions to a distal condylar region 38. The distal condylar region 38 transitions to a posterior chamfer region 40. A pair of posterior femoral condyles 42 form the posterior structure of the femoral component 12.

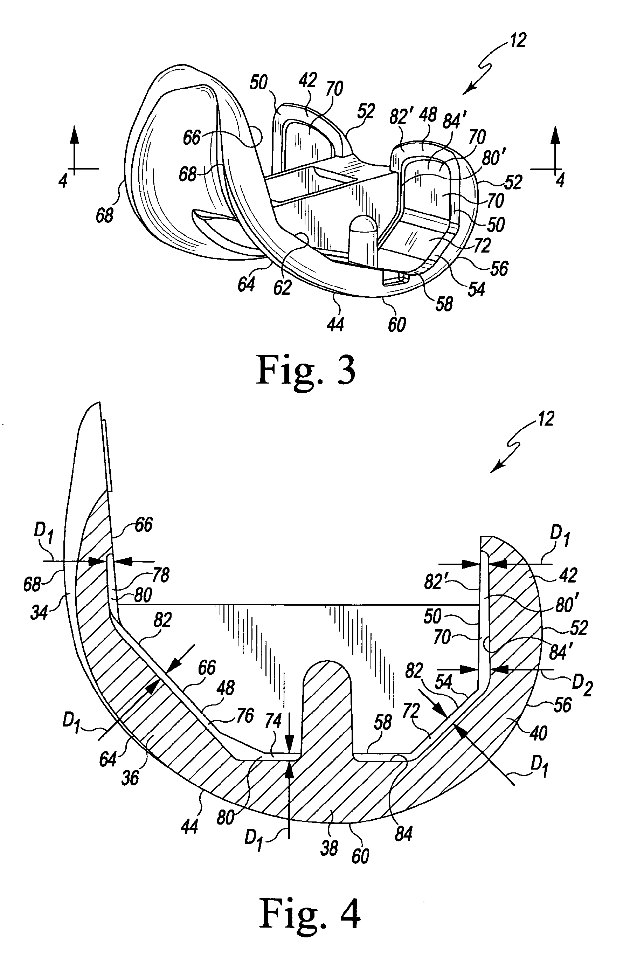

As shown in FIG. 2, both the lateral condyle surface 30 and the medial condyle surface 32 are formed in the articular side 44 of the femoral component 12. A fixation side 48 is opposite the articular side 44, and is the side of the femoral component 12 that contacts the surgically-prepared distal femur of the patient. The fixation side 48 includes multiple surfaces that mate with planar surfaces surgically cut into the patient's distal femur. Specifically, as shown in FIG. 3, a pair of posterior fixation surfaces 50 are opposite the posterior condyle surfaces 52, with one of the posterior fixation surfaces 50 being the medial fixation surface, the other the lateral fixation surface. As can be seen in FIG. 4, the posterior fixation surfaces 50 extend generally in the superior/inferior direction. A pair of distal fixation surfaces 58 (one being medially positioned, the other the laterally positioned) are opposite the distal condyle surfaces 60. The distal fixation surfaces 58 extend generally in the anterior/posterior direction. The medial and lateral posterior-chamfer fixation surfaces 54 are opposite the posterior-chamfer condyle surfaces 56. The medial and lateral posterior-chamfer fixation surfaces 54 extend superiorly and posteriorly from their respective medial and lateral distal fixation surfaces 58 in the direction toward their respective posterior fixation surfaces 50. The medial and lateral anterior-chamfer fixation surfaces 62 are opposite the anterior-chamfer condyle surfaces 64, respectively, and extend superiorly and anteriorly away from their respective distal fixation surfaces 58 in the direction toward an anterior fixation surface 66. The anterior fixation surface 66 is opposite the anterior condyle surface 68 and, like the posterior fixation surfaces 50, extends generally in the superior/inferior direction.

Each of the fixation surfaces has a cement pocket formed therein. In particular, a posterior cement pocket 70 is formed in each of the posterior fixation surfaces 50, a posterior-chamfer cement pocket 72 is formed in each of the posterior-chamfer fixation surfaces 54, a distal cement pocket 74 is formed in each of the distal fixation surfaces 58, an anterior-chamfer cement pocket 76 is formed in each of the anterior-chamfer fixation surfaces 62, and an anterior cement pocket 78 is formed in the anterior fixation surface 66. In the device described herein, the adjacent cement pockets are contiguous with one another such that a single, continuous cement pocket is formed in the fixation side 48 of the femoral component.

Each of the cement pockets 70, 72, 74, 76, 78 is formed by a sidewall 80 that extends away from a mounting rim 82. As can be seen in FIGS. 3 to 5, the sidewall 80 forms the perimeter of the respective cement pockets 70, 72, 74, 76, 78. A bottom wall 84 is spaced apart from the mounting rim 82 and is connected thereto by the sidewall 80. In such a way, the sidewall 80 and the bottom wall 84 collectively define the respective cement pockets 70, 72, 74, 76, 78.

The depth (D₁) of each of the posterior-chamfer cement pocket 72, the distal cement pocket 74, the anterior-chamfer cement pocket 76, and the anterior cement pocket 78 is approximately equal. In the device described herein, each of the cement pockets 72, 74, 76, and 78 is approximately 1mm deep (i.e., D₁ = 1mm).

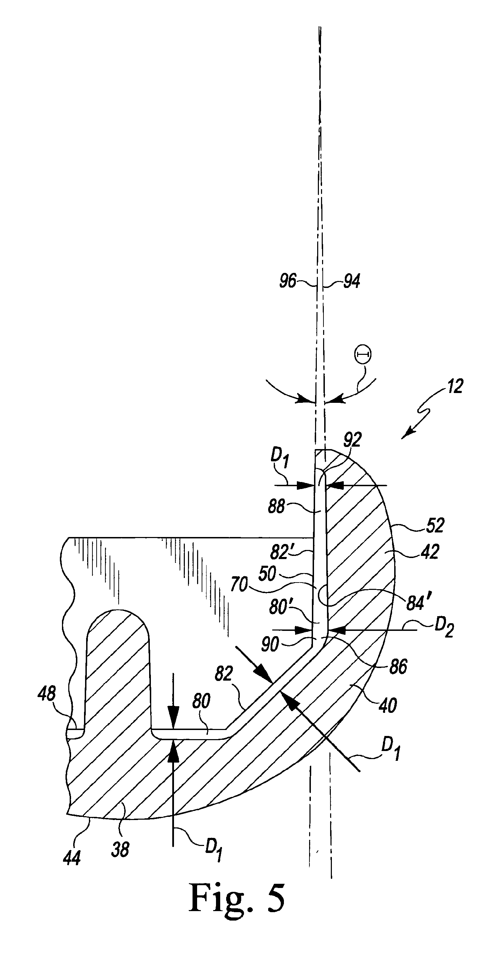

The posterior cement pocket 70, on the other hand, is angled and, as a result, is deeper at its inferior end than on its superior end. In particular, as shown in FIG. 5, the posterior cement pocket 70 is formed by a sidewall 80' that extends posteriorly from the mounting rim 82' of the posterior fixation surface 50 to the bottom wall 84'. When viewed sagittally, such as in the cross sectional view of FIGS. 4 and 5, the inferior end 86 of the sidewall 80' is wider than the superior end 88 of the sidewall 80'. As a result, the posterior cement pocket 70 is deeper at its inferior end 90 than at its superior end 92. In the device described herein, the superior end 92 of the posterior cement pocket 70 is approximately equal to the depth of the other cement pockets 72, 74, 76, and 78. In other words, the superior end 92 of the posterior cement pocket 70 is approximately 1mm deep (i.e., D₁ = 1mm). However, the inferior end 90 of the posterior cement pocket 70 is illustratively 1.5mm deep (i.e., D₂ = 1.5mm). It should be appreciated that other dimensions may also be used to fit the need of a given design of the femoral component 12.

Such an arrangement creates an angled bottom wall 84'. In particular, the bottom wall 84' slopes anteriorly from its inferior end 86 to its superior end 88. This sloped arrangement is illustratively shown in the cross sectional view of FIG. 5. As can be seen in that view, an imaginary plane 96 is defined by the mounting rim 82' of the posterior fixation surface 50, whereas the bottom wall 84' of the posterior fixation surface 50 defines an imaginary plane 94. The two imaginary planes 94, 96 form an acute angle (θ) therebetween. Such an acute angle is indicative of the slope of the bottom wall 84' relative to the mounting rim 82'.

During a surgical procedure to implant the femoral component 12 to the surgically-prepared distal end of the patient's femur, the cement pockets 70, 72, 74, 76, 78 are preloaded with bone cement. The femoral component 12 is then positioned on the patient's surgically-prepared distal femur, which has also been coated in bone cement. The angled arrangement of posterior cement pocket 70 hydraulically loads the bone cement within the cement pocket. This enhances containment of the bone cement and reduces the occurrences of cement plowing. The arrangement of the posterior cement pocket 70 also improves filling of the bone cement and pressurization which, in turn, leads to enhanced bonding of the femoral component 12 to the distal femur.

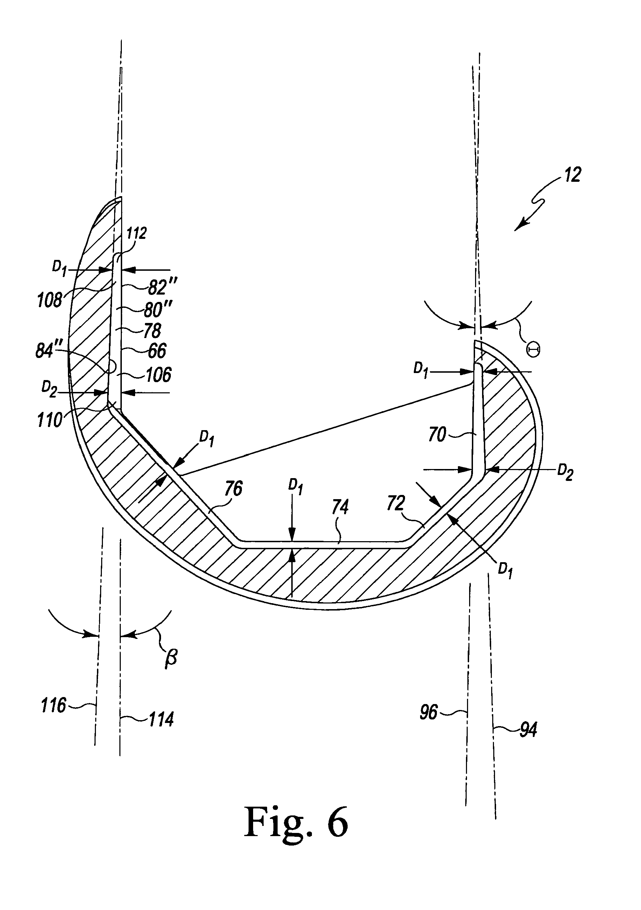

Referring now to FIG. 6, another femoral component 12 is shown. In this component, both the posterior cement pocket 70 and the anterior cement pocket 78 are angled. As such, both cement pockets 70, 78 are deeper at their respective inferior ends than at their respective superior ends. In the component shown in FIG. 6, the posterior cement pocket 70 is essentially the same as discussed above with reference to FIGS. 1 to 5. As to the anterior cement pocket 78, it is formed by a sidewall 80" that extends anteriorly from the mounting rim 82" of the anterior fixation surface 66 to the bottom wall 84". When viewed sagittally, such as in the cross sectional view of FIG. 6, the inferior end 106 of the sidewall 80" is wider than the superior end 108 of the sidewall 80". As a result, like the posterior cement pocket 70, the anterior cement pocket 78 is deeper at its inferior end 110 than at its superior end 112. In the device described herein, the superior end 112 of the anterior cement pocket 78 is approximately equal to the depth of the other cement pockets 72, 74, and 76. In other words, the superior end 112 of the anterior cement pocket 70 is approximately 1mm deep (i.e., D₁ = 1mm). However, the inferior end 110 of the anterior cement pocket 78 is illustratively 1.5mm deep (i.e., D₂ = 1.5mm). It should be appreciated that other dimensions may also be used to fit the need of a given design of the femoral component 12.

Such an arrangement creates an angled bottom wall 84". In particular, the bottom wall 84" of the anterior cement pocket 78 slopes posteriorly from its inferior end 106 to its superior end 108. This sloped arrangement is illustratively shown in FIG. 6 in which an imaginary plane 114 is defined by the mounting rim 82" of the anterior fixation surface 66, whereas the bottom wall 84" of the anterior fixation surface 66 defines an imaginary plane 116. The two imaginary planes 114, 116 form an acute angle (β) therebetween. Such an acute angle is indicative of the slope of the bottom wall 84" relative to the mounting rim 82".

During a surgical procedure to implant the femoral component 12 of FIG. 6 to the surgically-prepared distal end of the patient's femur, the cement pockets 70, 72, 74, 76, 78 are preloaded with bone cement. The femoral component 12 is then positioned on the patient's surgically-prepared distal femur, which has also been coated in bone cement. The angled arrangement of the cement pockets 70, 78 hydraulically loads the bone cement within the cement pockets 70, 78. This enhances containment of the bone cement and reduces the occurrences of cement plowing. This also improves filling of the bone cement and pressurization which, in turn, leads to enhanced bonding of the femoral component 12 to the distal femur.

It should be appreciated that although the component of FIG. 6 illustratively shows both the posterior cement pocket 70 and the anterior cement pocket 78 being angled, it should be appreciated that other constructions are also contemplated. For example, as shown in FIGS. 1 to 5, the femoral component 12 may be embodied with only the posterior cement pocket 70 being angled. Alternatively, the femoral component 12 may be embodied with only the anterior cement pocket 78 being angled.

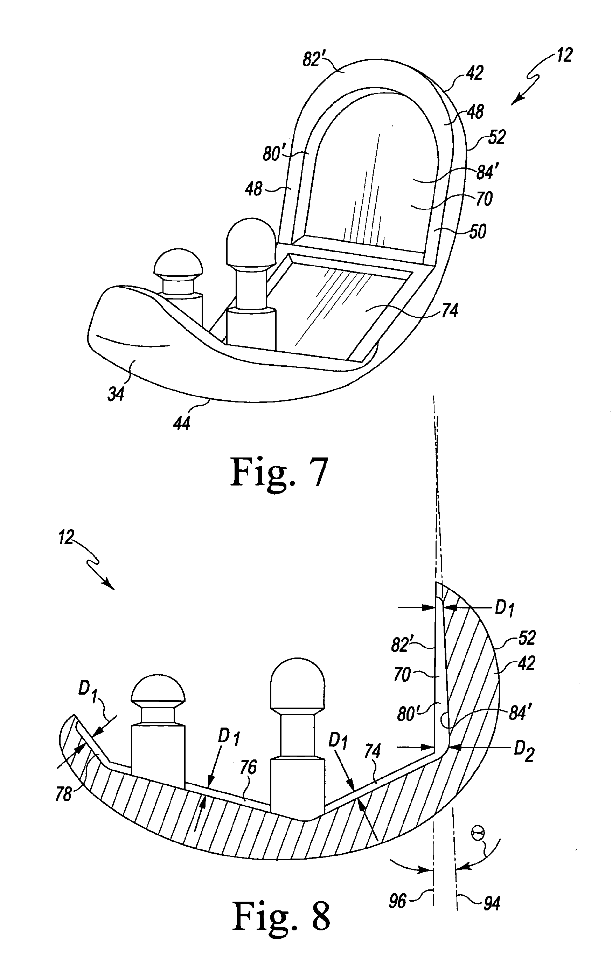

Referring now to FIGS. 7 and 8, the femoral component 12 may also be embodied as a unicompartmental femoral component. Like the other devices described herein, the posterior cement pocket 70 of the unicompartmental femoral component 12 may be angled. The angled arrangement of posterior cement pocket 70 hydraulically loads the bone cement within the cement pocket. This enhances containment of the bone cement and reduces the occurrences of cement plowing. The arrangement of the posterior cement pocket 70 also improves filling of the bone cement and pressurization which, in turn, leads to enhanced bonding of the unicompartmental femoral component 12 to the distal femur.

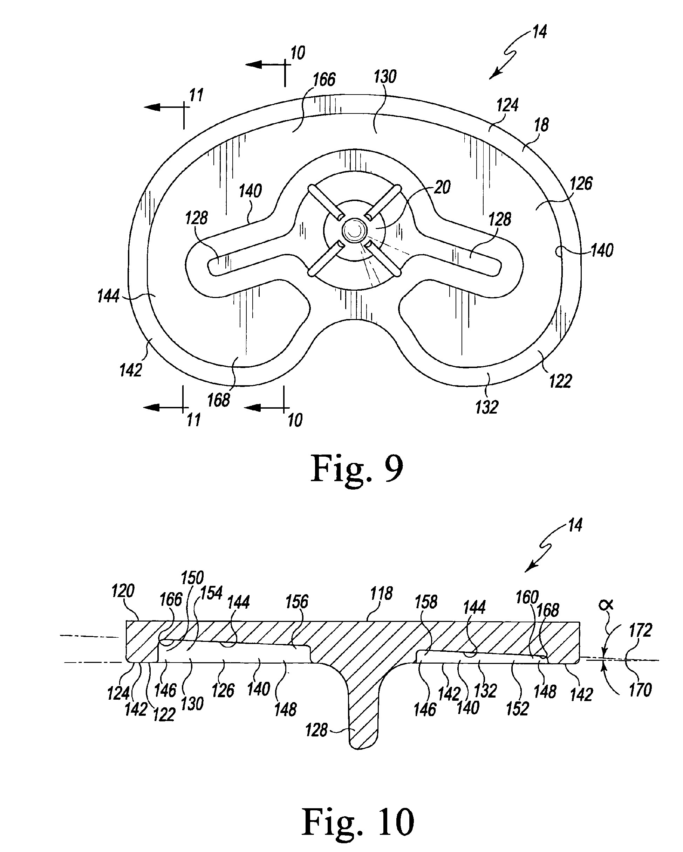

Referring now to FIGS. 9 to 11 which show the tibial tray 14 of FIGS. 1 and 2. As shown in FIG. 10, a flat planar surface 118 is formed in the superior side 120 of the tibial tray 14. Specifically, the superior surface 118 of the tibial tray's platform 18 defines a smooth planar surface on which the bearing 16 of the knee prosthesis 10 is free to rotate when the bearing's stem 22 (see FIG. 2) is positioned within the complementary bore 24 (see FIG. 2) of the tibial tray 14. In other devices, the superior surface 118 of the tibial tray's platform 18 may be embodied with one or more features on which the bearing 16 may be snap-fit or otherwise secured to the tibial tray 14. In such a way, the bearing 16 is fixed relative to the tibial tray 14 (i.e., it is not rotative or moveable in the anterior/ posterior or medial/lateral directions).

The tibial tray's inferior side 122 is opposite its superior side 120, and is the side of the tibial tray 14 that contacts the surgically-prepared proximal tibia of the patient. The elongated stem 20 extends inferiorly away from the tibial tray's inferior side 122. The tibial tray's inferior side 122 includes multiple surfaces that mate with planar surfaces surgically cut into the patient's proximal tibia. Specifically, as shown in FIGS. 9 and 10, an inferior fixation surface 124 extends generally in the transverse plane (i.e., in the direction of the anatomical transverse plane).

The inferior fixation surface 124 has a cement pocket 126 formed therein. In the device described herein, the tibial tray's fins 128 divide the cement pocket 126 into separate cement pockets at various locations along the tray's medial/lateral width. In particular, an anterior cement pocket 130 is positioned anteriorly of the tray's fins 128 with a posterior cement pocket 132 being positioned posteriorly of the tray's fins 128. However, since the fins 128 do not stretch to the medial and lateral edges of the tray 14, the cement pockets 130, 132 are contiguous with one another such that a single, continuous cement pocket 126 is formed in the inferior side 122 of the tibial tray 14. It should be appreciated; however, that the tibial tray 14 could be embodied with one or more separate cement pockets.

Each of the cement pockets 126, 130, 132 is formed by a sidewall 140 that extends superiorly away from a mounting rim 142. As can be seen in FIGS. 9 to 11, the sidewall 140 forms the perimeter of the respective cement pockets 126, 130, 132. A top wall 144 is spaced apart from the mounting rim 142 and is connected thereto by the sidewall 140. In such a way, the sidewall 140 and the top wall 144 collectively define the respective cement pockets 126, 130, 132.

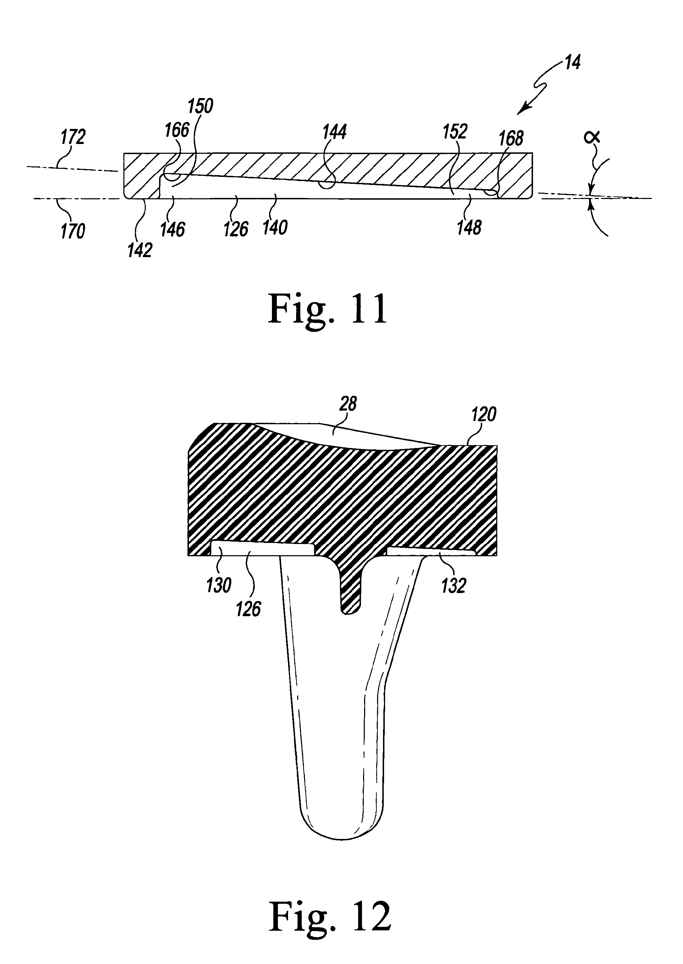

The cement pocket 126 is angled and, as a result, deeper at its anterior end than on its posterior end. In particular, when viewed sagittally, such as in the cross sectional view of FIGS. 10 and 11, the anterior end 146 of the sidewall 140 is wider than the posterior end 148 of the sidewall 140. As a result, the cement pocket 126 is deeper at its anterior end 150 than at its posterior end 152. Because the individual cement pockets formed by the fins 128 (i.e., the anterior cement pocket 130 and the posterior cement pocket 132) are, in essence, "sub-pockets" of the larger cement pocket 126, each of the cement pockets 130, 132 has a similar arrangement in which their respective anterior ends are deeper than their respective posterior ends. In particular, the anterior end 154 of the anterior cement pocket 130 is deeper than its posterior end 156. Likewise, the anterior end 158 of the posterior cement pocket 132 is deeper than its posterior end 160.

Such an arrangement creates an angled top wall 144. In particular, the top wall 144 slopes inferiorly from its anterior end 166 to its posterior end 168. This sloped arrangement is illustratively shown in the cross sectional views of FIGS. 10 and 11. As can be seen in that view, an imaginary plane 170 is defined by the mounting rim 142 of the inferior fixation surface 124, whereas the top wall 144 of the inferior fixation surface 124 defines an imaginary plane 172. The two imaginary planes 170, 172 form an acute angle (α) therebetween. Such an acute angle is indicative of the slope of the top wall 144 relative to the mounting rim 142.

During a surgical procedure to implant the tibial tray 14 to the surgically-prepared proximal end of the patient's tibia, the cement pocket 126 is preloaded with bone cement. The tibial tray 14 is then positioned on the patient's surgically-prepared proximal tibia, which has also been coated in bone cement. The angled arrangement of cement pocket 126 hydraulically loads the bone cement within the cement pocket. This enhances containment of the bone cement and reduces the occurrences of cement plowing. The arrangement of the cement pocket 126 also forces any excess bone cement out the anterior side of the tibial tray 14 where it can be readily wiped away or otherwise removed by the surgeon.

Referring now to FIG. 12, the tibial component may also be embodied as an all-poly tibial component. That is, a monolithic polymeric tibial component in lieu of a modular design in which the tibial tray and the bearing are embodied as separate components. In such a case, the lateral articular surface 26 and the medial articular surface 28 configured to articulate with a lateral condyle surface 30 and a medial condyle surface 32 of the femoral component 12, respectively, are formed in the superior side 120 of the tibial component. Like the tibial tray 14 described herein, the cement pocket 126 of the all-poly tibial component of FIG. 12 may be angled. The angled arrangement of cement pocket 126 hydraulically loads the bone cement within the cement pocket and forces any excess bone cement out the anterior side of the all-poly tibial component where it can be readily wiped away or otherwise removed by the surgeon.

## Claims

1. An implantable orthopaedic knee prosthesis (10), comprising a tibial component (14, 16) configured to be coupled to a surgically-prepared proximal tibia, the tibial component having:
(i) a superior side (120),
(ii) an inferior side (122) that is opposite the superior side, and
(iii) a fixation member (20) extending inferiorly from the inferior side, the inferior side of the tibial component comprising an inferior fixation surface (124) that extends generally in the transverse plane, in which the inferior fixation surface has a cement pocket (126) formed therein,
**characterised in that** the cement pocket is deeper at its anterior end (150) than at its posterior end (152).

2. The implantable orthopaedic prosthesis of claim 1, in which:
the inferior fixation surface (124) comprises a mounting rim (142) having a sidewall (140) extending superiorly therefrom,
the sidewall defines the perimeter of the cement pocket (126), and
when viewed sagittally, an anterior end (146) of the sidewall is wider than a posterior end (148) of the sidewall.

3. The implantable orthopaedic knee prosthesis of claim 1, further comprising a tibial bearing component (16), in which the tibial component comprises a tibial tray (14), and the tibial bearing component is positioned on the tibial tray.

4. The implantable orthopaedic knee prosthesis of claim 3, in which the tibial tray (14) is metallic and the tibial bearing (16) is polymeric.

5. The implantable orthopaedic knee prosthesis of claim 1, in which the superior side of the tibial component (14, 16) comprises a medial bearing surface (28) configured to articulate with the medial condyle surface of a femoral component, and a lateral bearing surface (26) configured to articulate with the lateral condyle surface of the femoral component.

6. The implantable orthopaedic knee prosthesis of claim 5, in which the tibial component comprises a monolithic polymeric component.

7. The implantable orthopaedic knee prosthesis of claim 1, which includes:
a femoral component (10) having a medial condyle surface (32) and a lateral condyle surface (30), and
a bearing (16) having (i) a medial bearing surface (28) configured to articulate with the medial condyle surface of the femoral component, and (ii) a lateral bearing surface (26) configured to articulate with the lateral condyle surface of the femoral component,
in which the tibial component is positioned below the bearing.

8. The implantable orthopaedic knee prosthesis of claim 7, in which the tibial tray (14) is metallic and the tibial bearing (16) is polymeric.

9. The implantable orthopaedic knee prosthesis of claim 7, in which the tibial bearing (16) is rotatable relative to the tibial tray (14).

10. The implantable orthopaedic knee prosthesis of claim 7, in which the tibial bearing (16) is fixed in position relative to the tibial tray (14).

## Patentansprüche

1. Implantierbare orthopädische Knieprothese (10), umfassend eine Schienbeinkomponente (14, 16), die konfiguriert ist, um mit einem chirurgisch vorbereiteten proximalen Schienbein gekoppelt zu werden, wobei die Schienbeinkomponente aufweist:
(i) eine obere Seite (120),
(ii) eine untere Seite (122), die sich gegenüber der oberen Seite befindet, und
(iii) ein Fixierungselement (20), das sich unterhalb der unteren Seite erstreckt, wobei die untere Seite der Schienbeinkomponente eine untere Fixierungsfläche (124) aufweist, die sich allgemein in der Querebene erstreckt, wobei die untere Fixierungsfläche eine darin ausgebildete Zementtasche (126) aufweist,
**dadurch gekennzeichnet, dass** die Zementtasche an ihrem vorderen Ende (150) tiefer als an ihrem hinteren Ende (152) ist.

2. Implantierbare orthopädische Prothese nach Anspruch 1, wobei:
die untere Fixierungsfläche (174) einen Befestigungsrand (142) mit einer sich davon nach oben erstreckenden Seiten (140) aufweist,
die Seitenwand den Umfang der Zementtasche (126) bildet und
bei sagittaler Betrachtung ein vorderes Ende (146) der Seitenwand breiter als ein hinteres Ende (148) der Seitenwand ist.

3. Implantierbare orthopädische Knieprothese nach Anspruch 1, ferner umfassend eine Schienbeinlagerkomponente (16), wobei die Schienbeinkomponente ein Tibiaplateau (14) aufweist und die Schienbeinlagerkomponente auf dem Tibiaplateau positioniert ist.

4. Implantierbare orthopädische Knieprothese nach Anspruch 3, wobei das Tibiaplateau (14) metallisch ist und das Schienbeinlager (16) polymer ist.

5. Implantierbare orthopädische Knieprothese nach Anspruch 1, wobei die obere Seite der Schienbeinkomponente (14, 16) eine mediale Lagerfläche (28), die konfiguriert ist, mit der medialen Kondylenfläche einer Oberschenkelkomponente ein Gelenk zu bilden, und eine laterale Lagerfläche (26) aufweist, die konfiguriert ist, um mit der lateralen Kondylenfläche der Oberschenkelkomponente ein Gelenk zu bilden.

6. Implantierbare orthopädische Knieprothese nach Anspruch 5, wobei die Schienbeinkomponente eine monolithische polymere Komponente aufweist.

7. Implantierbare orthopädische Knieprothese nach Anspruch 1, die enthält:
eine Oberschenkelkomponente (10) mit einer medialen Kondylenfläche (32) und einer lateralen Kondylenfläche (30), und
ein Lager (16) mit (i) einer medialen Lagerfläche (28), die konfiguriert ist, um mit der medialen Kondylenfläche der Oberschenkelkomponente ein Gelenk zu bilden, und (ii) einer lateralen Lagerfläche (26), die konfiguriert ist, um mit der lateralen Kondylenfläche der Oberschenkelkomponente ein Gelenk zu bilden,
wobei die Schienbeinkomponente unter dem Lager positioniert ist.

8. Implantierbare orthopädische Knieprothese nach Anspruch 7, wobei das Tibiaplateau (14) metallisch ist und das Schienbeinlager (16) polymer ist.

9. Implantierbare orthopädische Knieprothese nach Anspruch 7, wobei das Schienbeinlager (16) relativ zum Tibiaplateau (14) drehbar ist.

10. Implantierbare orthopädische Knieprothese nach Anspruch 7, wobei das Schienbeinlager (16) relativ zum Tibiaplateau (16) ortsfest ist.

## Revendications

1. Prothèse orthopédique implantable de genou (10) comprenant un composant tibial (14, 16) configuré pour être couplé à un tibia proximal préparé chirurgicalement, le composant tibial ayant :
(i) un côté supérieur (120),
(ii) un côté inférieur (122) qui est opposé au côté supérieur, et
(iii) un élément de fixation (20) s'étendant de manière inférieure par rapport au côté inférieur, le côté inférieur du composant tibial comprenant une surface de fixation inférieure (124) qui s'étend généralement dans le plan transversal, dans lequel la surface de fixation inférieure a une poche de ciment (126) formée à l'intérieur de cette dernière,
**caractérisée en ce que** la poche de ciment est plus profonde au niveau de son extrémité antérieure (150) qu'au niveau de son extrémité postérieure (152).

2. Prothèse orthopédique implantable selon la revendication 1, dans laquelle :
la surface de fixation inférieure (124) comprend un rebord de montage (142) ayant une paroi latérale (140) s'étendant de manière supérieure à partir de ce dernier,
la paroi latérale définit le périmètre de la poche de ciment (126), et
lorsqu'elle est observée de manière sagittale, l'extrémité antérieure (146) de la paroi latérale est plus large qu'une extrémité postérieure (148) de la paroi latérale.

3. Prothèse orthopédique implantable de genou selon la revendication 1, comprenant en outre un composant d'appui tibial (16) dans lequel le composant tibial comprend un plateau tibial (14), et le composant d'appui tibial est positionné sur le plateau tibial.

4. Prothèse orthopédique implantable de genou selon la revendication 3, dans laquelle le plateau tibial (14) est métallique et l'appui tibial (16) est polymère.

5. Prothèse orthopédique implantable de genou selon la revendication 1, dans laquelle le côté supérieur du composant tibial (14, 16) comprend une surface d'appui médiale (28) configurée pour s'articuler avec la surface condylienne médiale d'un composant fémoral et une surface d'appui latérale (26) configurée pour s'articuler avec la surface condylienne latérale du composant fémoral.

6. Prothèse orthopédique implantable de genou selon la revendication 5, dans laquelle le composant tibial comprend un composant polymère monolithique.

7. Prothèse orthopédique implantable de genou selon la revendication 1, comprenant :
un composant fémoral (10) ayant une surface condylienne médiale (32) et une surface condylienne latérale (30), et
un appui (16) ayant (i) une surface d'appui médiale (28) configurée pour s'articuler avec la surface condylienne médiale du composant fémoral et (ii) une surface d'appui latérale (26) configurée pour s'articuler avec la surface condylienne latérale du composant fémoral,
dans laquelle le composant tibial est positionné au-dessous de l'appui.

8. Prothèse orthopédique implantable de genou selon la revendication 7, dans laquelle le plateau tibial (14) est métallique et l'appui tibial (16) est polymère.

9. Prothèse orthopédique implantable de genou selon la revendication 7, dans laquelle l'appui tibial (16) peut tourner par rapport au plateau tibial (14).

10. Prothèse orthopédique implantable de genou selon la revendication 7, dans laquelle l'appui tibial (16) est fixé en position par rapport au plateau tibial (14).
